# EUROPEAN PATENT APPLICATION

(11) **EP 3 939 578 A1**
(43) Date of publication of application: **19.01.2022**
(21) Application number: 20185561.6
(22) Date of filing: 13.07.2020
(51) Int. Cl.: A61K 31/165, A61K 45/06, A61P 31/14, A61P 43/00, A61K 9/00

(54) **COMPOUNDS FOR TREATMENT OR PREVENTION OF AN INFECTION RESULTING FROM A CORONAVIRUS AND/OR A CORONAVIRUS-INDUCED DISEASE**

(71) Applicant: Novaremed Ltd., Petah Tikva, 4959382 (IL)
(72) Inventor: Kaplan, Eli, 4058 Basel (CH)
(74) Representative: Vossius & Partner Patentanwälte Rechtsanwälte mbB

(57) **Abstract**

The present invention relates to a compound of formula (I) or a salt or solvate thereof for use in the treatment or prevention of an infection resulting from a Coronavirus and/or a Coronavirus-induced disease and to the use thereof in the treatment or prevention of an infection resulting from a Coronavirus and/or a Coronavirus-induced disease, optionally in combination with other treatments.

## Description

The present invention relates to a compound of formula (I) or a salt or solvate thereof for use in the treatment or prevention of an infection resulting from a Coronavirus and/or a Coronavirus-induced disease, optionally in combination with other treatments, and to the use thereof in the treatment or prevention of an infection resulting from a Coronavirus and/or a Coronavirus-induced disease.

### Background

Compounds as described herein, such as 2-N(3-O-(propan-2-ol)-1-propyl-4-hydroxybenzene)-3-phenylpropylamide, have been disclosed in US 7,754,771 and medical uses thereof have been described in WO 2009/1099850, WO 2011/030105, US 2011/0086910 and WO 2013/084238.

Coronaviruses and Coronavirus-induced diseases have recently gained increasing attention with the outbreak of COVID-19 in 2019 and 2020. While most attention is directed at COVID-19, a long list of diseases can be caused by coronviruses. Coronaviruses are a group of related RNA viruses causing diseases in mammals and birds. In humans, respiratory tract infections are often observed, which may eventually be lethal to the subject. Particularly lethal varieties are coronaviruses leading to SARS, MERS, and the more recently observed COVID-19. As there are not yet any vaccines or antiviral drugs to prevent or treat human coronavirus infections, and in the face of a rapidly growing number of cases throughout the world, which lead to substantive detrimental effects not only on the health of the affected subjects but also on world economy, new antiviral drugs with activity against Coronaviruses and Coronavirus-induced diseases are urgently needed.

Coronaviruses (Orthocoronavirinae) are a subfamily of Coronaviridae. They are enveloped viruses with a positive-sense single-stranded RNA genome and a nucleocapsid of helical symmetry. At the start of an infection, the viral spike protein attaches to its complementary host cell receptor. After attachment, a protease of the host cell cleaves and activates the receptor-attached spike protein. Depending on the host cell protease available, cleavage and activation allows the virus to enter the host cell by endocytosis or direct fusion of the viral envelop with the host membrane (Antiviral Research. 2013, 100(3), pages 605 to 614).

### Summary of the invention

The present inventors found that compounds of formula (I) as described herein bind to the main protease of Covid-19 and related coronaviruses.

The present invention relates to a compound of formula (I) or a salt or solvate thereof for use in the treatment or prevention of an infection resulting from a Coronavirus and/or a Coronavirus-induced disease: 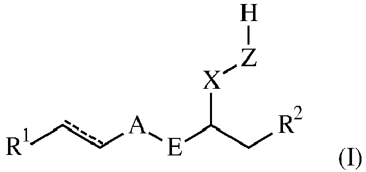 wherein
- -----: represents a single bond or a double bond;
- R¹: is selected from -CH₂OH, -CH₂O-alkyl, -CH(OH)CH₃, -CH(O-alkyl)CH₃, optionally substituted phenyl, optionally substituted indol-3-yl and optionally substituted imidazol-4-yl, wherein the optional substituent of the optionally substituted phenyl, the optionally substituted indol-3-yl and the optionally substituted imidazol-4-yl is independently selected from -OH and -O-alkyl,
- R²: is selected from optionally substituted phenyl, wherein the one or more optional substituents of the optionally substituted phenyl are selected from -halogen and -OH,
- A-E: is selected from -C(O)-N(H)-, -N(H)-C(O)-, -C(O)-CH₂-, -CH₂-N(H)-, -N(H)-CH₂- and -CH₂-C(O)-,
- X: is selected from -CH₂O-, -CH₂CH₂O-, -CH(CH₃)CH₂O- and -CH₂CH(CH₃)O-,
- Z: is selected from -CH₂CH₂O-, -CH(CH₃)CH₂O- and -CH₂CH(CH₃)O-.

The present invention furthermore concerns the use of a compound of formula (I) or a salt or solvate thereof as defined above for the treatment or prevention of an infection resulting from a Coronavirus and/or a Coronavirus-induced disease. In a further embodiment of the invention, the treatment or prevention of an infection resulting from a Coronavirus and/or a Coronavirus-induced disease is in combination with other treatments.

In addition, the present invention relates to a pharmaceutical composition for use in the treatment or prevention of an infection resulting from a Coronavirus and/or a Coronavirus-induced disease, said composition comprising a pharmaceutically effective amount of one or more of the compounds for use as defined above, optionally together with one or more pharmaceutically acceptable excipients. In one embodiment of the invention, the pharmaceutical composition may comprise one or more further pharmaceutically active compounds.

In a further aspect, the present invention concerns a method for treating or preventing an infection resulting from a Coronavirus and/or a Coronavirus-induced disease in a human or non-human animal patient in need thereof, wherein the method comprises administering to said patient a therapeutic effective amount of at least one compound as defined above or the pharmaceutical composition as defined above.

### Brief description of the figures

- Fig. 1:: X-ray powder diffraction pattern of polymorph 2
- Fig. 2:: SEM picture of polymorph 2

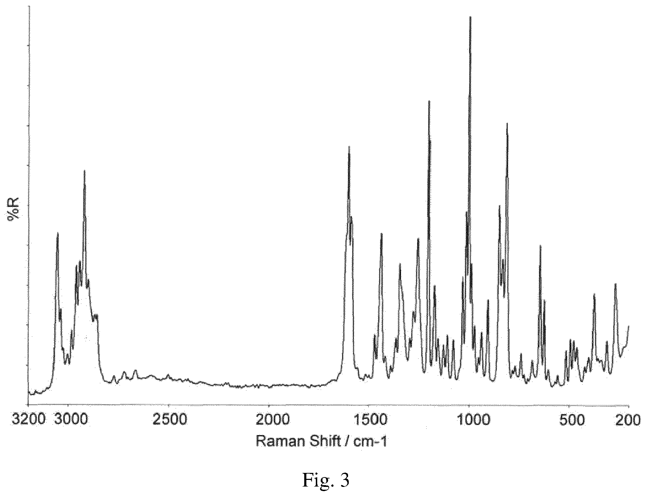

- Fig. 3:: Raman spectrum of polymorph 2, as measured on Bruker RFS 100/S at 1064 nm
- Fig. 4:: TGA/DTA data of polymorph 2

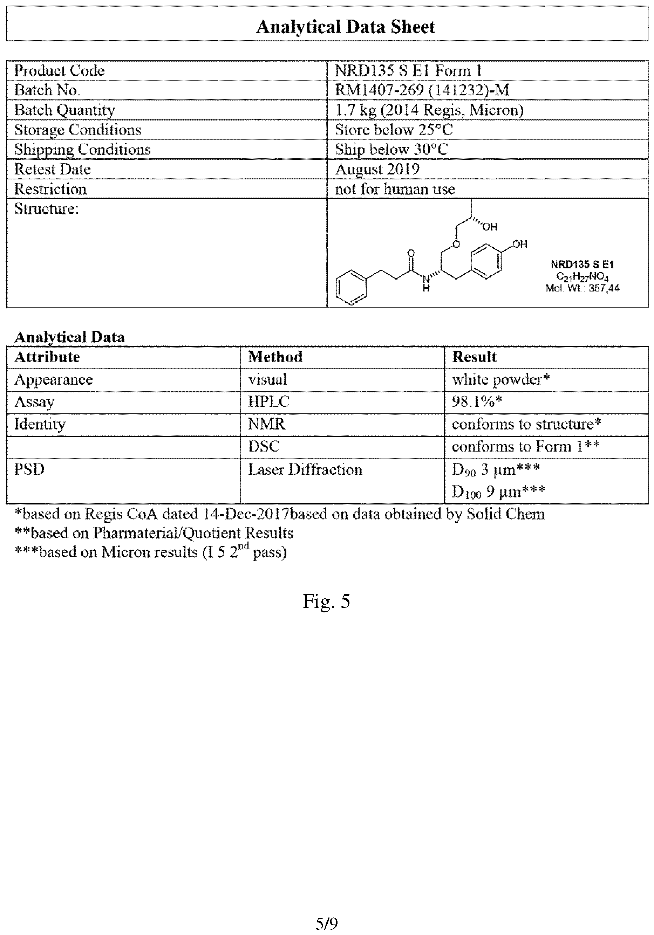

- Fig. 5: Analytical data sheet polymorph 1

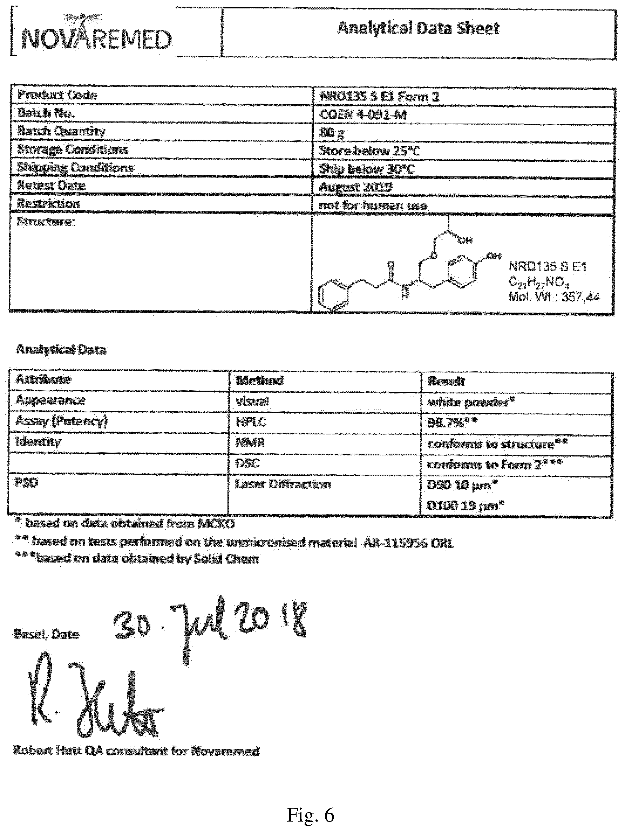

- Fig. 6: Analytical data sheet polymorph 2
- Fig. 7: 50ns MD with Desmond through Schrodinger in Pose in 6LU7
- Fig. 8: Analysis of expected interactions based on Fig. 7
- Fig. 9: Binding of NRD.E1 to the Covid-19 main protease

### Detailed description of the invention

The present invention relates to a compound of formula (I) or a salt or solvate thereof for use in the treatment or prevention of an infection resulting from a Coronavirus and/or a Coronavirus-induced disease: 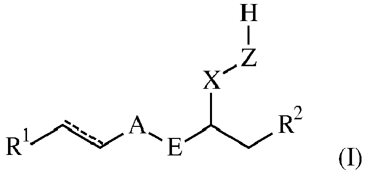 wherein
- -----: represents a single bond or a double bond;
- R¹: is selected from -CH₂OH, -CH₂O-alkyl, -CH(OH)CH₃, -CH(O-alkyl)CH₃, optionally substituted phenyl, optionally substituted indol-3-yl and optionally substituted imidazol-4-yl, wherein the optional substituent of the optionally substituted phenyl, the optionally substituted indol-3-yl and the optionally substituted imidazol-4-yl is independently selected from -OH and -O-alkyl,
- R²: is selected from optionally substituted phenyl, wherein the one or more optional substituents of the optionally substituted phenyl are selected from -halogen and -OH,
- A-E: is selected from -C(O)-N(H)-, -N(H)-C(O)-, -C(O)-CH₂-, -CH₂-N(H)-, -N(H)-CH₂- and -CH₂-C(O)-,
- X: is selected from -CH₂O-, -CH₂CH₂O-, -CH(CH₃)CH₂O- and -CH₂CH(CH₃)O-,
- Z: is selected from -CH₂CH₂O-, -CH(CH₃)CH₂O- and -CH₂CH(CH₃)O-.

In the examples of -A-E- it is to be understood that the left-hand side of the example corresponds to A and the right-hand side of the example corresponds to E. Thus, in the example "-C(O)-N(H)-", "-C(O)-" corresponds to A and "-N(H)-" corresponds to E.

In the examples of X, it is to be understood that the left bond shown as "-" is connected to the carbon atom below X as shown in formula (I) and the right bond shown as "-" is connected to Z as shown in formula (I).

In the examples of Z, it is to be understood that the left bond shown as "-" is connected to X as shown in formula (I) and the right bond shown as "-" is connected to the "H" as shown to be connected to Z in formula (I). It is furthermore to be understood that the "H" as shown to be connected to Z in formula (I) is connected to Z in such a manner that it is part of a hydroxyl group. In other words, if Z is -CH(CH₃)CH₂O-, then Z-H is -CH(CH₃)CH₂OH.

It is preferred that ----- represents a single bond.

R¹ is preferably selected from optionally substituted phenyl, optionally substituted indol-3-yl and optionally substituted imidazol-4-yl, wherein the optional substituent of the optionally substituted phenyl, the optionally substituted indol-3-yl and the optionally substituted imidazol-4-yl is independently selected from -OH and -O-alkyl. More preferably, R¹ is selected from optionally substituted phenyl, wherein the optional substituent of the optionally substituted phenyl independently selected from -OH and -O-alkyl. Even more preferably R¹ is phenyl.

R² is preferably selected from optionally substituted phenyl, wherein the one or more optional substituents of the optionally substituted phenyl are selected from -halogen and -OH. More preferably, R² is 4-hydroxy-phenyl which may be further substituted with one or two halogen(s).

A-E is preferably selected from -C(O)-N(H)-, -C(O)-CH₂- and -CH₂-N(H)-. More preferably, A-E is selected from -C(O)-N(H)- and -C(O)-CH₂-. Alternatively, A-E is more preferably selected from -C(O)-N(H)- and -CH₂-N(H)-. Most preferably, A-E is -C(O)-N(H)-.

X is preferably selected from -CH₂O- and -CH₂CH₂O-. More preferably, X is -CH₂O-.

Z is preferably selected from -CH(CH₃)CH₂O- and -CH₂CH(CH₃)O-. More preferably, Z is -CH₂CH(CH₃)O-.

It is furthermore preferred that the compound of formula (I) is a compound of formula (II) or a salt or solvate thereof:

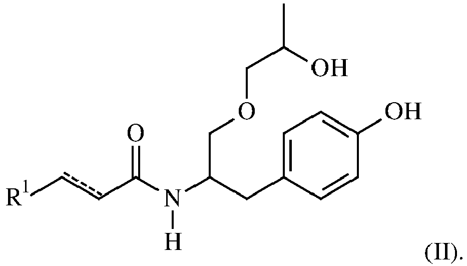

It is to be understood that the compound of formula (I) and the compound of formula (II) may be present in the form of two enantiomers and even in the form of four diastereomers. Some compounds of formula (I) or some compounds of formula (II) may even exist as more than four stereoisomers.

In the compounds of the present invention, it preferred that the stereo configuration at the chiral carbon atom next to group E, in particular next to E being NH, be *S*.

Accordingly, the compound of formula (I) is preferably a compound of formula (IIa) or a salt or solvate thereof:

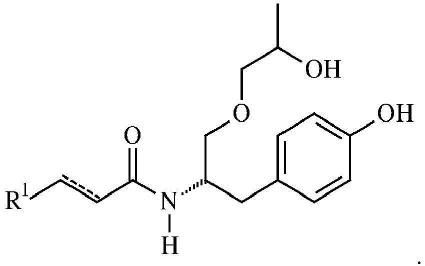

This compound can preferably be selected from 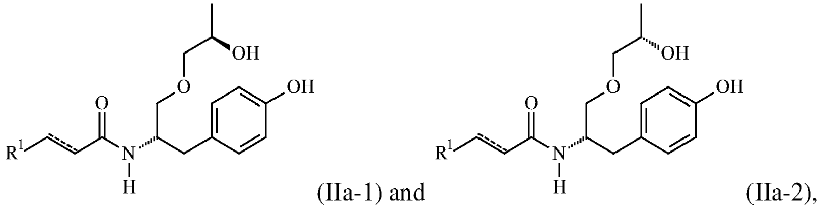 among which the compound of formula (IIa-2) is more preferred.

In other examples of the present invention, the compound of formula (I) is a compound of formula (IIb) or a salt or solvate thereof: 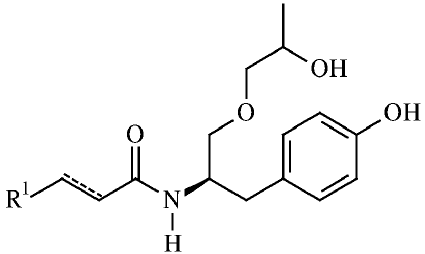 which preferably selected from

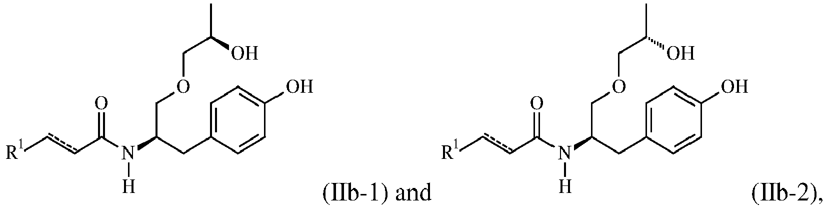

It is to be understood that the definitions of ----- and R¹ as set out herein apply not only to formula (I) but also to any examples thereof, such as formulae (II), (IIa), (IIb), (IIa-1), (IIa-2), (IIb-1), (IIb-2).

More preferably, the compound used in the present invention is a compound of the following chemical formula:

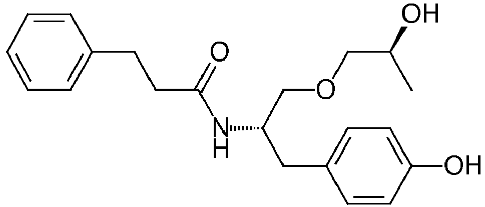

This compound ((*S*,*S*)-2-N(3-O-(propan-2-ol)-1-propyl-4-hydroxybenzene)-3-phenylpropylamide), including its enantiomers and diastereomers may be prepared as described in WO 2013/084238, which is incorporated herein by reference, particularly Examples 1 and 2 thereof.

The ((*S*,*S*)-2-N(3-O-(propan-2-ol)-1-propyl-4-hydroxybenzene)-3-phenylpropylamide) is an enantiomer that is preferably substantially free of other stereoisomeric forms of this compound. A representative substantially pure enantiomer comprises greater than 90% by weight of one enantiomer of the compound and less than 10% by weight of the other stereoisomeric forms of the compound, preferably greater than 95% by weight of one enantiomer of the compound and less than 5% by weight of the other stereoisomeric forms of the compound, even more preferably greater than 98% by weight of one enantiomeric form of the compound and less than 2% by weight of the other stereoisomeric forms of the compound. The term "other stereoisomeric forms" typically refers to the (S,R), (R,S) and (R,R) enantiomeric or diastereomeric forms of the compound 2-N(3-O-(propan-2-ol)-1-propyl-4-hydroxybenzene)-3-phenylpropylamide.

A polymorph of this compound is preferably in the present invention. This polymorph, hereinafter also referred to polymorph 2, may be characterized by methods such as X-ray, DSC and/or Raman spectroscopy.

The present inventors have surprisingly found polymorph 2 to be less hygroscopic and more stable than other crystal forms, such as polymorph 1 as discussed later. Moreover, polymorph 2 was surprisingly found to have a better bioavailability as compared to polymorph 1. This is particularly surprising in view of its higher stability. Thermal analysis of polymorph 2 shows a melting point with an onset at 104.5°C and a melting point peak at 107.2°C. The melting enthalpy ΔHₘₑₗₜ was about 121 J/g.

The thermal analysis for determining the melting point and melting enthalpy of the claimed polymorph may be conducted using a TGA/DTA analyzer (such as Perkin-Elmer STA 600 TGA/DTA analyzer) at 25 °C temperature, heating the sample at a rate of 10°C/min, typically from 25°C to 300°C, during which time the change in weight is monitored as well as the differential thermal analysis (DTA) signal, while the purge gas used is nitrogen at a flow rate of 20 cm³/min.

Scanning electron Microscopy (SEM) indicates that polymorph 2 has a regular rectangular block habit with particles ranging in size from 2 to 10 µm long and 2 to 5 µm wide. The particle size is typically expressed as the D₅₀ volume median particle diameter as measured using laser diffraction.

Gravimetric Vapour Sorption (GVS) shows that polymorph 2 is only slightly hygroscopic with a 0.6% weight gain to 80% RH.

Polymorph 2 of the invention may be isolated by preparing a slurry in water and crystallizing from a 50/50 mixture by weight of methanol and water. It may also be prepared by rapid cooling of a solution in chloroform and isopropyl acetate.

Polymorph 2 of the invention typically has an X-ray powder diffraction pattern (CuKa) comprising a peak at 19.0 ± 0.2 °2θ, more preferably 19.0 ± 0.1 °2θ and even more preferably 19.0 ± 0.05 °2θ. More preferably, the polymorphic form has an X-ray powder diffraction pattern (CuKa) further comprising one or more peaks selected from peaks at 11.25 ± 0.2, 17.38 ± 0.2, 17.57 ± 0.2, 20.74 ± 0.2, 20.91 ± 0.2, 22.42 ± 0.2 and 23.30 ± 0.2 °2θ. The further peaks are even more preferably selected from 11.25 ± 0.1, 17.38 ± 0.1, 17.57 ± 0.1, 20.74 ± 0.1, 20.91 ± 0.1, 22.42 ± 0.1 and 23.30 ± 0.1 °2θ. Still more preferably, the peaks are selected from 11.25 ± 0.05, 17.38 ± 0.05, 17.57 ± 0.05, 20.74 ± 0.05, 20.91 ± 0.05, 22.42 ± 0.05 and 23.30 ± 0.05 °2θ. Preferably, the peak at 19.0 ± 0.2 °2θ, more preferably, at 19.0 ± 0.1 °2θ, is the peak with the highest relative intensity in the X-ray powder diffraction pattern (CuKa) of polymorph 2 (in particular within the range of 5 to 40 °2θ).

Polymorph 2 preferably does not contain any peaks within the ranges of 15 ± 0.3 °2θ and 16.5 ± 0.2 °2θ. More preferably, polymorph 2 does not contain any peaks within the ranges of 15 ± 0.5 °2θ and 16.5 ± 0.3 °2θ.

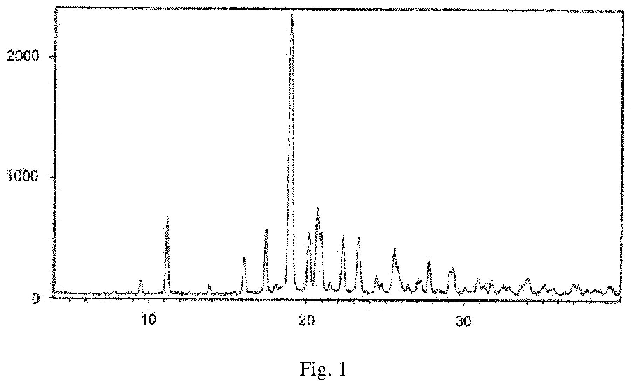

Even more preferably, the X-ray powder diffraction pattern (CuKa) of Polymorph 2 of the invention is substantially as shown in FIG. 1.

Polymorph 2 typically melting point in the range from about 105 °C to about 110 °C. More preferably, the melting point is in the range from about 106 °C to about 109 °C, preferably about 107 °C to about 108 °C.

In addition to the above, it has even more surprisingly been found in dog bioavilability tests, as described in the experimental data described herein, that the bioavailability of polymorph 2 of the invention is exceptionally high, and in particular higher than the bioavailability of polymorph 1.

In contrast, the characteristics of polymorph 1 indicate a higher hygroscopicity and lower stability than the inventive polymorph 2.

Namely, thermal analysis of polymorph 1 shows a melting point with an onset at 119.6°C and a melting point peak at 125.6°C. The melting enthalpy ΔHₘₑₗₜ was about 19 J/g.

Simultaneous Thermal analysis (STA) data of polymorph 1 further indicates a small weight loss of just under 1% between 90 and 115°C followed by the melt with onset at ∼ 119.5°C in a single thermal event. Polymorph 1 may thus be a partial hydrate.

Scanning electron Microscopy (SEM) indicates that polymorph 1 has mostly irregular shaped particles less than 10 µm across wide.

Gravimetric Vapour Sorption (GVS) shows that polymorph 1 is hygroscopic with a weight increase of ∼ 1.3% to 70% RH. However, a rapid moisture uptake then follows between 70% and 80% RH to 5.6% and there is hysteresis (gap) between the adsorption and desorption cycles which hinted towards form change. This different form appeared to be more hygroscopic, with a second adsorption cycle showing a weight increase well above a satisfactory limit of 2% at an RH of just 40%.

Polymorph 1 may be prepared by recrystallizing from a variety of solvents, such as methanol, ethanol, 2-propanol, acetonitrile, acetone, 1,4-dioxane and dimethyl formamide.

It is believed that the effects of the present invention may also be achieved if R¹ is selected from carbocyclyl and heterocyclyl as defined herein. Accordingly, the present invention also relates to compounds of formula (I) wherein R¹ is selected from alkyl which is optionally substituted with one or more -halogen and/or -hydroxy and/or -O-alkyl groups, optionally substituted carbocyclyl and optionally substituted heterocyclyl, wherein the optional substituent of the optionally substituted carbocyclyl and the optionally substituted heterocyclyl is/are independently selected from alkyl, -C(O)-alkyl, -halogen, -CF₃, -CN,-COOR*, -OR*, -(CH₂)_{q}NR*R*, -C(O)-NR*R* and -NR*-C(O)-C₁₋₆ alkyl, wherein each R* is independently selected from -H and alkyl and q is 0 to 4.

The present invention furthermore concerns the use of a compound of formula (I) or a salt or solvate thereof as defined above for the treatment or prevention of an infection resulting from a Coronavirus and/or a Coronavirus-induced disease.

In addition, the present invention relates to a pharmaceutical composition for use in the treatment or prevention of an infection resulting from a Coronavirus and/or a Coronavirus-induced disease, said composition comprising a pharmaceutically effective amount of one or more of the compounds for use as defined above, optionally together with one or more pharmaceutically acceptable excipients.

In a further aspect, the present invention concerns a method for treating or preventing an infection resulting from a Coronavirus and/or a Coronavirus-induced disease in a human or non-human animal patient in need thereof, wherein the method comprises administering to said patient a therapeutic effective amount of at least one compound as defined above or the pharmaceutical composition as defined above.

The following definitions apply throughout the present specification, unless specifically indicated otherwise.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₆ alkyl" denotes an alkyl group having 1 to 6 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₆ alkyl, more preferably C₁₋₄ alkyl, even more preferably to methyl or ethyl, and still more preferably to methyl.

As used herein, the term "alkylene" refers to an alkanediyl group, i.e. a divalent saturated acyclic hydrocarbon group which may be linear or branched. A "C₁₋₆ alkylene" denotes an alkylene group having 1 to 6 carbon atoms, and the term "C₀₋₃ alkylene" indicates that a covalent bond (corresponding to the option "C₀ alkylene") or a C₁₋₃ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl. The number of carbon atoms in the carbocyclyl group is not particularly limited and is preferably 3 to 14, more preferably 3 to 7.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. Unless defined otherwise, "heterocyclyl" preferably refers to heteroaryl, heterocycloalkyl or heterocycloalkenyl. The number of carbon atoms in the carbocyclyl group is not particularly limited and is preferably 5 to 14, preferably 5 to 10.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), anthracenyl, or phenanthrenyl. Unless defined otherwise, an "aryl" preferably has 5 to 14 ring atoms, more preferably 5 to 10 ring atoms, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromenyl, xanthenyl, phenoxathiinyl, pyrrolyl (e.g., 2H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolizinyl, isoindolyl, indolyl (e.g., 3H-indolyl), indazolyl, purinyl, isoquinolyl, quinolyl, phthalazinyl, naphthyridinyl, quinoxalinyl, cinnolinyl, pteridinyl, carbazolyl, beta-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, furazanyl, phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzoxazolyl, benzisoxazolyl, benzimidazolyl, 1H-tetrazolyl, 2H-tetrazolyl, coumarinyl, or chromonyl. Unless defined otherwise, a "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, or adamantyl. Unless defined otherwise, "cycloalkyl" preferably refers to a C₃₋₁₄ cycloalkyl, and more preferably refers to a C₃₋₇ cycloalkyl. A particularly preferred "cycloalkyl" is a monocyclic saturated hydrocarbon ring having 3 to 7 ring members.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). "Heterocycloalkyl" may, e.g., refer to oxetanyl, tetrahydrofuranyl, piperidinyl, piperazinyl, aziridinyl, azetidinyl, pyrrolidinyl, imidazolidinyl, morpholinyl (e.g., morpholin-4-yl), pyrazolidinyl, tetrahydrothienyl, octahydroquinolinyl, octahydroisoquinolinyl, oxazolidinyl, isoxazolidinyl, azepanyl, diazepanyl, oxazepanyl or 2-oxa-5-aza-bicyclo[2.2.1]hept-5-yl. Unless defined otherwise, "heterocycloalkyl" preferably refers to a 3 to 14 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₄ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms and carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. "Heterocycloalkenyl" may, e.g., refer to 1,2,3,6-tetrahydropyridinyl. Unless defined otherwise, "heterocycloalkenyl" preferably refers to a 3 to 14 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-Cl), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

A "solvate" refers to an association or complex of one or more solvent molecules and the compound of formula (I). Examples of solvents that form solvates include, but are not limited to, water, isopropanol, ethanol, methanol, dimethyl sulfoxide (DMSO), ethyl acetate, acetic acid, acetonitril, and ethanolamine. The term "hydrate" refers to the complex where the solvent molecule is water. It is to be understood that such solvates of the compounds of the formula (I) also include solvates of pharmaceutically acceptable salts of the compounds of the formula (I).

A "cocrystal" refers to a crystalline structure that contains at least two different compounds that are solid in their pure form under ambient conditions. Cocrystals are made from neutral molecular species, and all species remain neutral after crystallization; further, typically and preferably, they are crystalline homogeneous phase materials where two or more building compounds are present in a defined stoichiometric ratio. See hereto Wang Y and Chen A, 2013; and Springuel GR, et al., 2012; and US Patent 6,570,036.

A skilled person will appreciate that the substituent groups comprised in the compounds of formula (I) may be attached to the remainder of the respective compound via a number of different positions of the corresponding specific substituent group. Unless defined otherwise, the preferred attachment positions for the various specific substituent groups are as illustrated in the examples.

As used herein, any reference to a compound of formula (I) is to be understood as equally well applying to any specific examples of a compound of formula (I), such as compounds of formulae (II), (IIa), (IIb), (IIa-1), (IIa-2), (IIb-1) and (IIb-2).

The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, inter alia, comprise curative treatment (e.g. disease modifying, preferably leading to a complete response and eventually to healing of the disorder or disease) and palliative treatment (including symptomatic relief).

The term "prevention" or "prophylaxis" of a disorder or disease as used herein is also well known in the art. For example, a patient/subject suspected of being prone to suffer from a disorder or disease may particularly benefit from a prevention of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" or "prophylaxis" comprises the use of the polymorph of the present invention before any clinical and/or pathological symptoms are diagnosed or determined or can be diagnosed or determined by the attending physician.

As used herein, the term "treatment or prevention of an infection resulting from a Coronoavirus and/or a Coronavirus-induced disease" indicates that not only the infection as such may be treated or prevented but also side-effects and long-term effects standing in relationship with such an infection may be treated or prevented using the compound of the invention.

As used herein, the term "a Coronavirus and/or a Coronavirus-induced disease" relates to a virus of the genus α-CoV, β-CoV, γ-CoV or δ-CoV. In a particular embodiment of the invention the Coronavirus is selected from the group consisting of Human coronavirus OC43 (HCoV-OC43), Human coronavirus HKU1 (HCoV-HKU1), Human coronavirus 229E (HCoV-229E), Human coronavirus NL63 (HCoV-NL63, New Haven coronavirus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019"). As such, the present invention relates to the prevention or treatment of known coronaviruses. However, the compound of the invention may also be used to prevent or treat newly occurring viruses of the coronavirus family.

In a particular embodiment of the invention, the Coronavirus-induced disease is selected from the group consisting of Middle East Respiratory Syndrome (MERS), Severe Acute Respiratory Syndrom (SARS) and COVID-19.

The term "about" preferably refers to ±10% of the indicated numerical value, more preferably to ±5% of the indicated numerical value, and in particular to the exact numerical value indicated. For example, the expression "about 100" preferably refers to 100 ±10% (i.e., 90 to 110), more preferably to 100 ±5% (i.e., 95 to 105), and even more preferably to the specific value of 100. If the term "about" is used in connection with the endpoints of a range, it preferably refers to the range from the lower endpoint 10% of its indicated numerical value to the upper endpoint +10% of its indicated numerical value, more preferably to the range from of the lower endpoint 5% to the upper endpoint +5%, and even more preferably to the range defined by the exact numerical values of the lower endpoint and the upper endpoint. Thus, the expression "about 10 to about 20" preferably refers to the range of 9 to 22, more preferably to the range of 9.5 to 21, and even more preferably to the range of 10 to 20. If the term "about" is used in connection with the endpoint of an open-ended range, it preferably refers to the corresponding range starting from the lower endpoint 10% or from the upper endpoint +10%, more preferably to the range starting from the lower endpoint 5% or from the upper endpoint +5%, and even more preferably to the open-ended range defined by the exact numerical value of the corresponding endpoint. For example, the expression "at least about 10%" preferably refers to at least 9%, more preferably to at least 9.5%, and even more preferably to at least 10%.

The term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

Any parameters referred to herein (including, e.g., any amounts/concentrations indicated in "mg/ml" or in "% (v/v)", and any pH values) are preferably to be determined at standard ambient temperature and pressure conditions, particularly at a temperature of 25°C (298.15 K) and at an absolute pressure of 1 atm (101.325 kPa).

It is to be understood that the present invention specifically relates to each and every combination of features and embodiments described herein, including any combination of general and/or preferred features/embodiments.

In this specification, a number of documents including patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### Pharmaceutically acceptable salts etc.

The scope of the invention embraces all pharmaceutically acceptable salt forms of the compounds of formula (I) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compounds of formula (I) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) is a hydrochloride salt. Accordingly, it is preferred that the compound of formula (I), including any one of the specific compounds of formula (I) described herein, is in the form of a hydrochloride salt, a hydrobromide salt, a mesylate salt, a sulfate salt, a tartrate salt, a fumarate salt, an acetate salt, a citrate salt, or a phosphate salt, and it is particularly preferred that the compound of formula (I) is in the form of a hydrochloride salt.

Furthermore, the compounds of formula (I) may exist in the form of different isomers, in particular stereoisomers (including, e.g., geometric isomers (or cis/trans isomers), enantiomers and diastereomers) or tautomers. All such isomers of the compounds of formula (I) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds provided herein.

The scope of the invention also embraces compounds of formula (I), in which one or more atoms are replaced by a specific isotope of the corresponding atom. For example, the invention encompasses compounds of formula (I), in which one or more hydrogen atoms (or, e.g., all hydrogen atoms) are replaced by deuterium atoms (i.e., ²H; also referred to as "D"). Accordingly, the invention also embraces compounds of formula (I) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) can be increased using deuteration techniques known in the art. For example, a compound of formula (I) or a reactant or precursor to be used in the synthesis of the compound of formula (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

The present invention also embraces compounds of formula (I), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers or imaging probes in positron emission tomography (PET). The invention thus includes (i) compounds of formula (I), in which one or more fluorine atoms (or, e.g., all fluorine atoms) are replaced by ¹⁸F atoms, (ii) compounds of formula (I), in which one or more carbon atoms (or, e.g., all carbon atoms) are replaced by ¹¹C atoms, (iii) compounds of formula (I), in which one or more nitrogen atoms (or, e.g., all nitrogen atoms) are replaced by ¹³N atoms, (iv) compounds of formula (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula (I), in which one or more bromine atoms (or, e.g., all bromine atoms) are replaced by ⁷⁷Br atoms, (vii) compounds of formula (I), in which one or more iodine atoms (or, e.g., all iodine atoms) are replaced by ¹²⁰I atoms, and (viii) compounds of formula (I), in which one or more iodine atoms (or, e.g., all iodine atoms) are replaced by ¹²⁴I atoms. In general, it is preferred that none of the atoms in the compounds of formula (I) are replaced by specific isotopes.

### Formulation, dosage forms etc.

As detailed above, the compounds provided herein may be administered as compounds *per se* or may be formulated as medicaments, e.g. as pharmaceutical composition or combination. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers, or any combination thereof.

In particular, the pharmaceutical compositions may comprise one or more solubility enhancers, such as, e.g., poly(ethylene glycol), including poly(ethylene glycol) having a molecular weight in the range of about 200 to about 5,000 Da, ethylene glycol, propylene glycol, non-ionic surfactants, tyloxapol, polysorbate 80, macrogol-15-hydroxystearate, phospholipids, lecithin, dimyristoyl phosphatidylcholine, dipalmitoyl phosphatidylcholine, distearoyl phosphatidylcholine, cyclodextrins, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, carboxyalkyl thioethers, hydroxypropyl methylcellulose, hydroxypropylcellulose, polyvinylpyrrolidone, vinyl acetate copolymers, vinyl pyrrolidone, sodium lauryl sulfate, dioctyl sodium sulfosuccinate, or any combination thereof.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

The pharmaceutical compositions can be formulated by techniques known to the person skilled in the art, such as the techniques published in "Remington: The Science and Practice of Pharmacy", Pharmaceutical Press, 22nd edition. The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I) or the above described pharmaceutical compositions comprising a compound of formula (I) may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, and vaginal.

If said compounds or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

Alternatively, said compounds or pharmaceutical compositions can be administered in the form of a suppository or pessary, or it may be applied topically in the form of a gel, hydrogel, lotion, solution, cream, ointment or dusting powder. The compounds of the present invention may also be dermally or transdermally administered, for example, by the use of a skin patch.

Said compounds or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides (see, e.g., US 3,773,919), copolymers of L-glutamic acid and gamma-ethyl-L-glutamate (Sidman, U. et al., Biopolymers 22:547-556 (1983)), poly(2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP133988). Sustained-release pharmaceutical compositions also include liposomally entrapped compounds. Liposomes containing a compound of the present invention can be prepared by methods known in the art, such as, e.g., the methods described in any one of: DE3218121; Epstein et al., Proc. Natl. Acad. Sci. (USA) 82:3688-3692 (1985); Hwang et al., Proc. Natl. Acad. Sci. (USA) 77:4030-4034 (1980); EP0052322; EP0036676; EP088046; EP0143949; EP0142641; JP 83-118008; US 4,485,045; US 4,544,545; and EP0102324.

Said compounds or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the compounds of formula (I) for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to the emulsification/spray drying process disclosed in WO 99/16419 or WO 01/85136. Spray drying of solution formulations of the compounds of the present invention can be carried out, e.g., as described generally in the "Spray Drying Handbook", 5th ed., K. Masters, John Wiley & Sons, Inc., NY (1991), and in WO 97/41833 or WO 03/053411.

For topical application to the skin, said compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

The present invention thus relates to the compounds or the pharmaceutical compositions provided herein, wherein the corresponding compound or pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route. Particularly preferred routes of administration of the compounds or pharmaceutical compositions of the present invention are oral forms of administration.

Typically, a physician will determine the dosage which will be most suitable for an individual subject. The specific dose level and frequency of dosage for any particular individual subject may be varied and will depend upon a variety of factors including the age, body weight, general health, sex, diet, mode and time of administration, rate of excretion, drug combination, the severity of the particular condition, and the individual subject undergoing therapy.

A proposed, yet non-limiting dose of the compounds according to the invention for administration to a human (of approximately 70 kg body weight) may be 0.05 to 2000 mg, preferably 0.1 mg to 1000 mg, of the active ingredient per unit dose. The unit dose may be administered, e.g., 1, 2, 3 or more times per day. The unit dose may also be administered 1 to 7 times per week, e.g., with one, two or more administration(s) per day. It will be appreciated that it may be necessary to make routine variations to the dosage depending on the age and weight of the patient/subject as well as the severity of the condition to be treated. The precise dose and also the route of administration will ultimately be at the discretion of the attendant physician.

The compounds of formula (I) can be used in combination with other therapeutic agents, including in particular other antiviral agents, preferably other anti-coronavirus agents. When a compound of the invention is used in combination with a second therapeutic agent active against the same disease, the dose of each compound may differ from that when the compound is used alone. The combination of a compound of the present invention with a second therapeutic agent may comprise the administration of the second therapeutic agent simultaneously/concomitantly or sequentially/separately with the compound of the invention.

### Examples

Examples of the present invention are given purely for illustrative and non-limiting purposes.

### Example 1: Docking of 4 diastereomers of 2-N(3-O-(propan-2-ol)-1-propyl-4-hydroxybenzene)-3-phenylpropylamide) in Covid-19 protease structures

The protein structures of the main Covid-19 protease were gathered and prepared by assigning atom types, adding hydrogens, optimizing hydrogen bonding and removing waters. Ligands of the targets are covalent binders and were detached. Ligands in structures were used as reference points for the creation of docking grids. Dockings were carried out on 35 structures taking in those with the highest resolution, most diverse structural conformations and most similar ligands and sampling some of the other structures. Resultant poses were initially assessed by score to identify those that warranted further attention Poses with the most similar conformation were assessed further.

There are 136 structures of the main protease of Covid-19 in the Protein DataBank. Dockings were carried out in Maestro on 35 structures to identify trends. In general those structures containing larger peptide ligands gave the best poses.

Poses were generated with another tool, MOE and the results showed similar binding modes. This provides additional validation that this is a probable binding mode since MOE uses a different forcefield to calculate interaction energies

The largest factor driving ligand binding is the displacement of water from high energy environments into low energy bulk water. The Watermap tool from Schrodinger can be used to approximate the contribution of water displacement to binding using a series of short Molecular Dynamics runs.

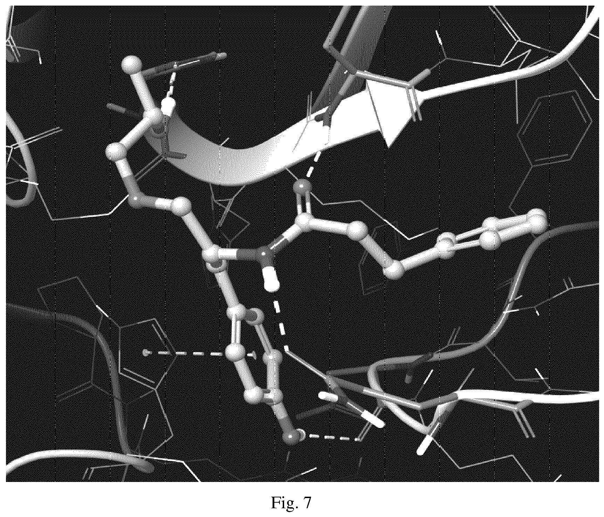

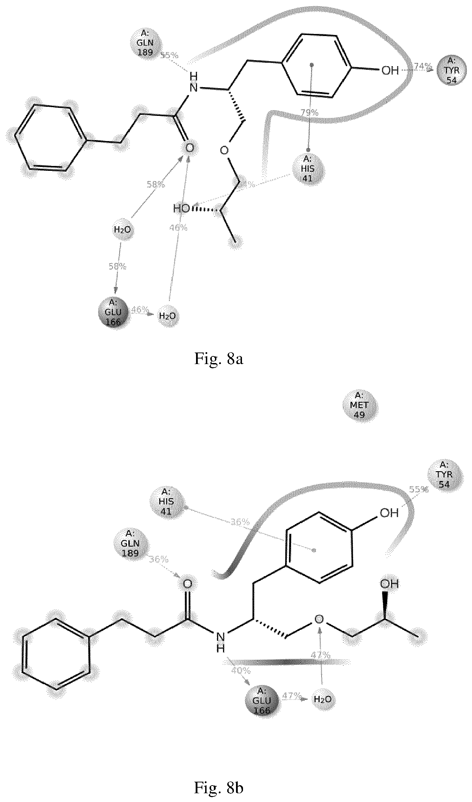

To gain confidence a several short Molecular Dynamics runs were performed. The results showed the peripheral hydrogen bonding to H163,Q189 and Q166 to be transient. 50ns MD with Desmond through Schrodinger in Pose in 6LU7 are shown in Figure 7, with an analysis of expected interactions being shown Figures 8a and 8b.

A pose image was validated as a reasonable pose with similar poses generated using Glide (Schrodinger software) and MOE (Chemical Computing Group). Binding of E1 to the Covid-19 main protease (M^{pro}) is highly probable based on the favorable binding modes generated through docking showing a good fit of E1 in the substrate pocket of M^{pro} in which N3 binds in PDBs 6LU7 and 7BQY.

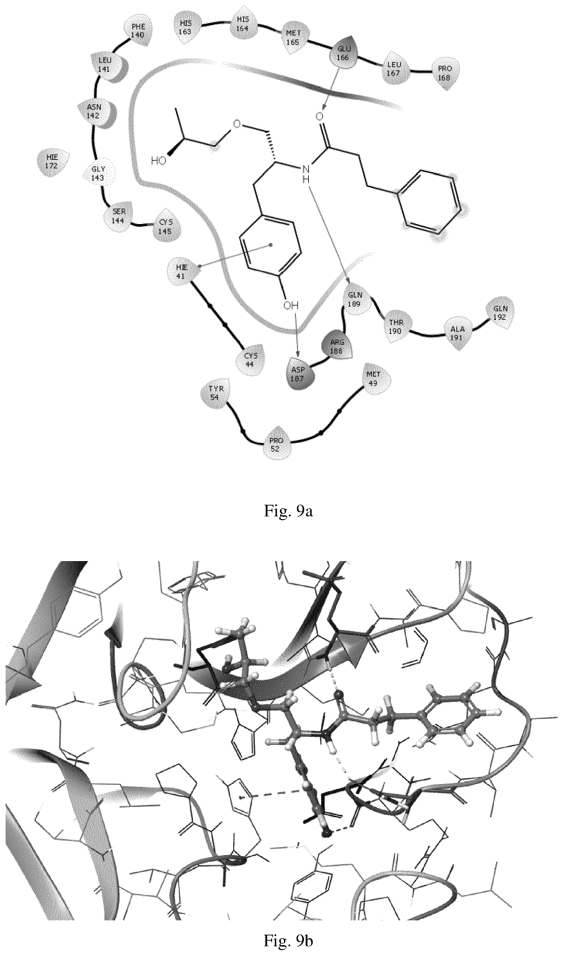

The most promising enantiomer was identified and named as E1, whose chemical structure is shown in Fig. 9a. In particular for the relaxed conformer, good hydrogen bonding show promise. Considering the stereo centers, that of the alcohol protects the H163 hydrogen bond from solvent by the methyl tail placement and the central stereo center orients the three vectors in line with the pocket shape. This can be seen from Figures 9a and 9b.

### Example 2 - Preparation of (S,S)-2-N(3-O-(propan-2-ol)-1-propyl-4-hydroxybenzene)-3-phenylpropylamide

(S,S)-2-N(3-O-(propan-2-ol)-1-propyl-4-hydroxybenzene)-3-phenylpropylamide was prepared as described in WO 2013/084238 and US 2011/0086910.

In a first step, 2 g of methyl lactate was reacted with excess of benzyl bromide to get 880 mg of (S)-benzyloxymethyl lactate. The reaction was performed by slurring sodium hydride in THF and cooling down to approximately -15°C. The reaction mixture was then allowed to warm slowly to room temperature and stirred for approximately 1 to 2 hours. The reaction was quenched with saturated ammonium chloride solution and extracted with MTBE twice followed by the removal of solvent on a rotary evaporator to obtain a crude oil. The crude product was purified by column chromatography to yield pure (S)-2-benzyloxymethyl lactate. The (R)-2-benzyloxymethyl lactate isomer was present at 0.93% only. The yield of this step may be increased by avoiding the presence of moisture in the reaction solution.

In a second step, 880 mg (S)-2-benzyloxymethyl lactate obtained in step 1 were reduced using lithium aluminum hydride to obtain (S)-2-benzyloxypropylene glycol in 83.8% yield with 98.7% purity. A solution of pure (S)-2-benzyloxymethyl lactate in methylene chloride was stirred and a solution of lithium aluminum hydride was slowly added thereto at approximately 5°C. The reaction was monitored by TLC and quenched by USP-PW water very carefully. No racemization occurred in this step.

In a third step, the (S)-2-benzyloxypropylene glycol was then reacted with methane sulfonyl chloride in methylene chloride in the presence of triethyl amine to yield the mesylate in 88% yield. A solution of step 2 was stirred in methylene chloride and methane sulfonyl chloride was added to it dropwise at <5°C. After the addition was complete, the progress of the reaction was monitored by TLC. The reaction was quenched with USP-PW water. After the layers were separated, the aqueous layer was back extracted with methylene chloride. The methylene chloride layers were then combined and washed with USP-PW water 3 times to remove most of the methane sulfonic acid. No racemization occurred in this step.

In a fourth step, the mesylate (of step 3) was coupled with S-O-benzyl tyrosinol to form the bis-protected product in 22.7% yield, with a purity of 97.4%. The reaction was carried out at room temperature using a combination of DMF as the solvent and sodium hydride as the base. The reaction went to completion after stirring for at least 12 hours at room temperature.

In a fifth step, 340 mg of the product of step 4 were reduced by hydrogenation in the presence of 10% palladium on carbon catalyst and hydrochloric acid using methylene chloride as a solvent at 50°C. The reaction went to completion in approximately 4 hours with no racemization to yield the desired product in 84.3% yield and 98.9% purity. More specifically, the catalyst was removed by filtration and the filtrate was then concentrated at 33°C. The resulting mixture of solid and oil was mixed with ethyl acetate. The resulting slurry was filtered and the solids washed with ethyl acetate and dried under vacuum at 40 to 45°C to obtain the desired product.

### Example 3 - Preparation of Polymorph 2

(S,S)-2-N(3-O-(propan-2-ol)-1-propyl-4-hydroxybenzene)-3-phenylpropylamide (500 mg) was suspended in water (5 mL) and the resulting suspension was shaken and temperature-cycled between 40°C and 25°C every 4 hours for 72 hours. Excess water was decanted off as far as practicably possible using a syringe and needle. Thereafter, the product was dried, initially by evaporation of the water at ambient temperature then at 50°C under vacuum until a constant weight was achieved.

### Method use for analysis of the polymorphs

### X-ray Powder Diffraction (XRPD)

Approximately 5 mg of sample was gently compressed on the XRPD zero back ground single obliquely cut silica sample holder. The sample was then loaded into a Philips X-Pert MPD diffractometer and analysed using the following experimental conditions.
Tube anode: Cu
Generator tension: 40 kV
Tube current: 40 mA
Wavelength alpha1: 1.5406 Å
Wavelength alpha2: 1.5444 Å
Start angle [2 theta]: 5
End angle [2 theta]: 50
Continuous scan
Representative X-ray data was acquired at a slower scan speed over a range of 4 - 40°2Θ.

### Raman spectroscopy

### Raman

Raman spectra were acquired on a Bruker RFS 100/S utilizing excitation wavelength of 1064 nm. The samples were prepared for analysis by placing the material in a sample holder and positioning this in the spectrometer.

### Simultaneous Thermal Analysis (STA)

Simultaneous thermal analysis (STA) in this context refers to the simultaneous application of thermogravimetry (TGA) and differential thermal analysis (DTA) to one and the same sample in a single instrument.
Approximately 5 mg of sample was accurately weighed into a ceramic crucible and it was placed into the chamber of Perkin-Elmer STA 600 TGA/DTA analyzer at ambient (25°C) temperature. The sample was then heated at a rate of 10°C/min, typically from 25°C to 300°C, during which time the change in weight was monitored as well as DTA signal. The purge gas used was nitrogen at a flow rate of 20 cm³/min.

### Differential scanning calorimetry (DSC)

DSC was investigated with a Netzsch-DSC 204 F1 Phoenix. Approximately 5-6 mg of the sample was placed into a DSC pan. The analysis was performed in a sealed aluminum pan with a pinhole. The sample was heated under nitrogen atmosphere from 25 to 125 °C at a rate of 10 K/min.

### Scanning Electron Microscopy (SEM)

Scanning Electron Microscopy was performed using a Tescan Vega3 Scanning Electron Microscope operating at 15KV and a slight tilt of 15°.
Prior to analysis samples were prepared by adhering the sample to an SEM stub using double sided carbon impregnated sticky tabs supplied by Agar Scientific.
The prepared stubs were then coated with 15 nm gold using a Quorum Q150ES sputter coater.

### Gravimetric Vapour Sorption (GVS)

Approximately 10 mg of sample was placed into a wire-mesh vapour sorption balance pan and loaded into an 'IgaSorp' vapour sorption balance (Hiden Analytical Instruments). The sample was then dried by maintaining a 0% humidity environment until no further weight change was recorded. Subsequently, the sample was subjected to a ramping profile from 0 - 90 % RH at 10 % RH increments, maintaining the sample at each step until equilibration had been attained (99% step completion). Upon reaching equilibration, the % RH within the apparatus was ramped to the next step and the equilibration procedure repeated. After completion of the sorption cycle, the sample was then dried using the same procedure. The weight change during the sorption/desorption cycles were then monitored, allowing for the hygroscopic nature of the sample to be determined.

### Results of the analysis of the polymorphs

The XRPD obtained was as shown in Fig. 1. In the following, a listing of the observed peaks is provided.

**Table 1**

| Position [°2θ] | Height [cts] | FWHM Left [°2θ] | d-spacing [Å] | Relative Intensity [%] |
|---|---|---|---|---|
| 7.8142 | 64.76 | 0.2342 | 11.31424 | 1.77 |
| 9.5751 | 148.88 | 0.1004 | 9.23703 | 4.06 |
| 11.0922 | 494.90 | 0.1171 | 7.97688 | 13.49 |
| 11.2534 | 729.20 | 0.1171 | 7.86298 | 19.88 |
| 13.8655 | 94.91 | 0.2007 | 6.38697 | 2.59 |
| 16.1232 | 400.23 | 0.1673 | 5.49737 | 10.91 |
| 17.3820 | 830.84 | 0.1004 | 5.10197 | 22.65 |
| 17.5766 | 705.98 | 0.1004 | 5.04592 | 19.24 |
| 18.0595 | 115.54 | 0.1673 | 4.91207 | 3.15 |
| 19.0182 | 3668.47 | 0.1840 | 4.66658 | 100.00 |
| 20.1171 | 642.72 | 0.1171 | 4.41407 | 17.52 |
| 20.7352 | 1113.40 | 0.1338 | 4.28387 | 30.35 |
| 20.9093 | 1063.71 | 0.2676 | 4.24859 | 29.00 |
| 21.5697 | 321.51 | 0.1673 | 4.11997 | 8.76 |
| 22.2235 | 624.43 | 0.1338 | 4.00023 | 17.02 |
| 22.4170 | 690.54 | 0.1338 | 3.96613 | 18.82 |
| 23.3016 | 816.32 | 0.3011 | 3.81753 | 22.25 |
| 24.4333 | 232.43 | 0.1338 | 3.64321 | 6.34 |
| 25.5555 | 658.66 | 0.2342 | 3.48572 | 17.95 |
| 26.0885 | 221.55 | 0.1004 | 3.41571 | 6.04 |
| 26.5053 | 161.46 | 0.2007 | 3.36294 | 4.40 |
| 26.9953 | 190.19 | 0.1338 | 3.30299 | 5.18 |
| 27.3243 | 244.20 | 0.2342 | 3.26397 | 6.66 |
| 27.7524 | 485.75 | 0.1338 | 3.21458 | 13.24 |
| 27.9287 | 392.11 | 0.1004 | 3.19470 | 10.69 |
| 28.4146 | 111.14 | 0.2676 | 3.14115 | 3.03 |
| 29.0727 | 481.13 | 0.1004 | 3.07154 | 13.12 |
| 29.3470 | 502.21 | 0.1673 | 3.04345 | 13.69 |
| 30.1336 | 141.80 | 0.2676 | 2.96577 | 3.87 |
| 30.8642 | 283.90 | 0.1673 | 2.89721 | 7.74 |
| 31.3612 | 180.65 | 0.1673 | 2.85243 | 4.92 |
| 31.8212 | 261.34 | 0.2342 | 2.81223 | 7.12 |
| 32.5372 | 211.67 | 0.2342 | 2.75197 | 5.77 |
| 32.8345 | 156.37 | 0.1673 | 2.72772 | 4.26 |
| 34.1862 | 251.13 | 0.2676 | 2.62290 | 6.85 |
| 35.0957 | 192.69 | 0.1673 | 2.55699 | 5.25 |
| 35.6799 | 147.37 | 0.1673 | 2.51645 | 4.02 |
| 36.9814 | 236.11 | 0.2007 | 2.43082 | 6.44 |
| 37.2854 | 169.91 | 0.2342 | 2.41170 | 4.63 |
| 37.8147 | 117.77 | 0.1338 | 2.37915 | 3.21 |
| 38.3576 | 129.23 | 0.2676 | 2.34672 | 3.52 |
| 39.2479 | 193.48 | 0.1338 | 2.29551 | 5.27 |

¹H-NMR spectra were measured for (S,S)-2-N(3-O-(propan-2-ol)-1-propyl-4-hydroxybenzene)-3-phenylpropylamide before and after crystallization to obtain polymorph 2. These spectra where consistent with each other, indicating that no chemical conversions had occured.

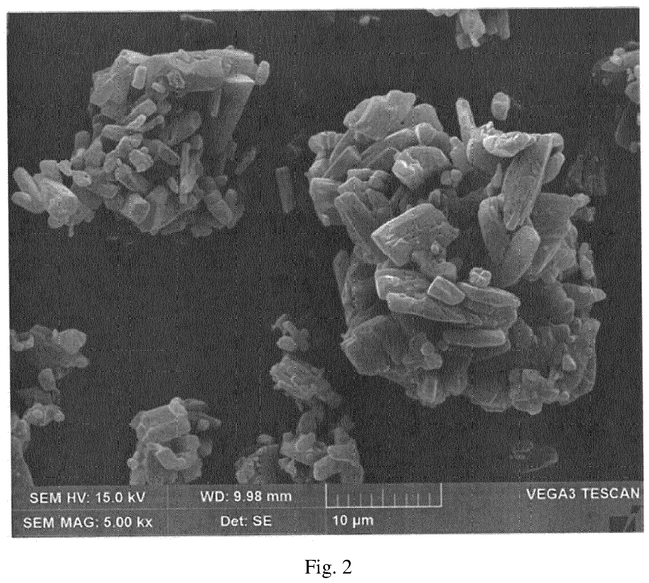

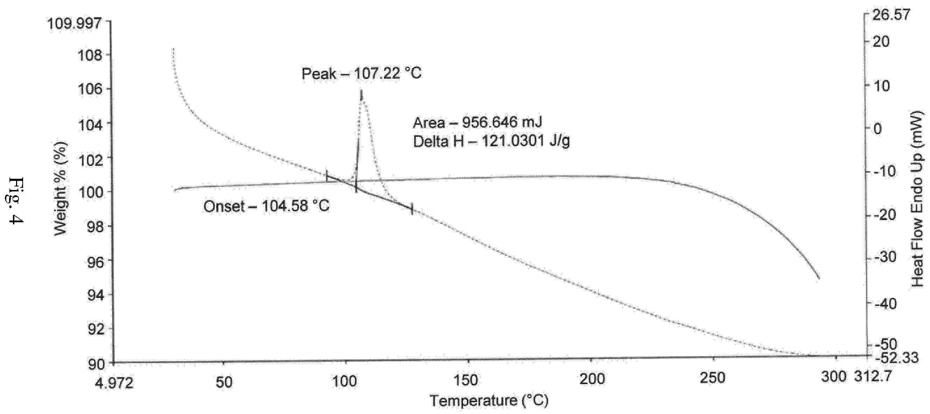

The STA data showed no weight loss, which shows that the sample was not hydrated or solvated. The DTA thermogram (cf. Fig.4) shows a sharp melt with onset at about 104.5 °C. Scanning electron Microscopy (SEM) (cf. Fig 2) indicated this form had a regular rectangular block habit with particle diameters ranging from 2 to 10 µm length and 2 to 5 µm width.

GVS showed that the sample was only slightly hygroscopic with a 0.6% weight gain to 80%RH which indicates Form 2 was less hygroscopic than Form 1. There was a sharp weight loss at 90% RH for both cycles which may have been the result of further crystallisation of amorphous content. This resulted in an overall weight loss of ∼ 1% by the end of the two adsorption/desorption cycles, but XRPD indicated no change of polymorphic form.

The bioavailability of polymorphs 1 and 2 has also been tested and made available in PCT/EP2020/051528 as Example 3 and is incorporated herein by reference. Accordingly, it was found that the bioavailability of polymorphs 2 is higher than for polymorph 1.

## Claims

1. A compound of formula (I) or a salt or solvate thereof for use in the treatment or prevention of an infection resulting from a Coronavirus and/or a Coronavirus-induced disease, optionally in combination with other treatments: 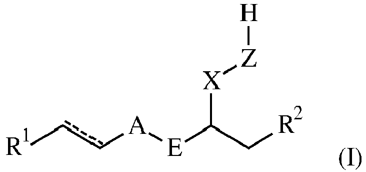 wherein
----- represents a single bond or a double bond;
R¹ is selected from -CH₂OH, -CH₂O-alkyl, -CH(OH)CH₃, -CH(O-alkyl)CH₃, optionally substituted phenyl, optionally substituted indol-3-yl and optionally substituted imidazol-4-yl, wherein the optional substituent of the optionally substituted phenyl, the optionally substituted indol-3-yl and the optionally substituted imidazol-4-yl is independently selected from -OH and -O-alkyl,
R² is selected from optionally substituted phenyl, wherein the one or more optional substituents of the optionally substituted phenyl are selected from -halogen and -OH,
A-E is selected from -C(O)-N(H)-, -N(H)-C(O)-, -C(O)-CH₂-, -CH₂-N(H)-, -N(H)-CH₂- and -CH₂-C(O)-,
X is selected from -CH₂O-, -CH₂CH₂O-, -CH(CH₃)CH₂O- and -CH₂CH(CH₃)O-,
Z is selected from -CH₂CH₂O-, -CH(CH₃)CH₂O- and -CH₂CH(CH₃)O-.

2. The compound for use according to claim 1, wherein the compound of formula (I) is selected from a compound of formula (Ia): 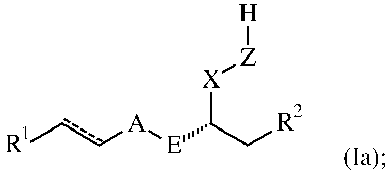 or
wherein the compound of formula (I) is selected from a compound of formula (Ib):

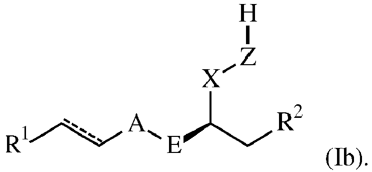

3. The compound for use according to any one of claims 1 to 2, wherein R¹ is selected from optionally substituted phenyl, optionally substituted indol-3-yl and optionally substituted imidazol-4-yl.

4. The compound for use according to any one of claims 1 to 3, wherein R¹ is selected from optionally substituted phenyl, preferably phenyl.

5. The compound for use according to any one of claims 1 to 4, wherein R² is selected from phenyl having an -OH group in para position and optionally having one or two further substituents selected from -halogen and -OH.

6. The compound for use according to any one of claims 1 to 5, wherein R² is phenyl having an -OH group in para position.

7. The compound for use according to any one of claims 1 to 6, wherein A-E is -C(O)-N(H)-.

8. The compound for use according to any one of claims 1 to 7, wherein compound of formula (I) is a compound of formula (II) or a salt or solvate thereof:

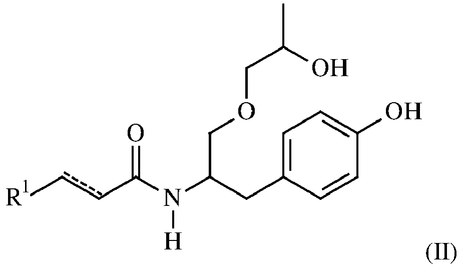

9. The compound for use according to any one of claims 1 to 8, wherein the compound of formula (I) is a compound of formula (IIa) or a salt or solvate thereof: 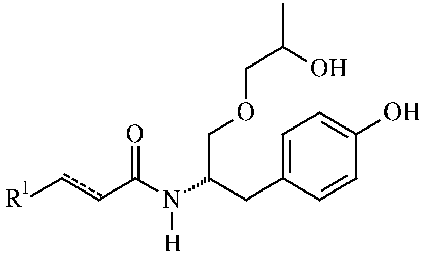 preferably selected from 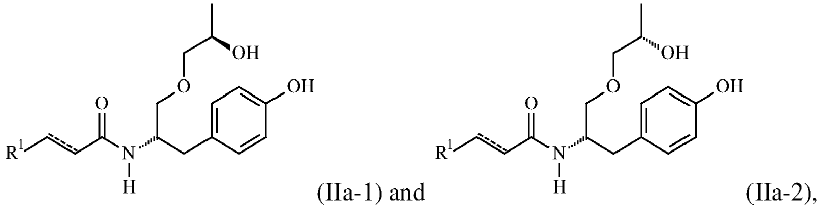 wherein the definitions of ----- and R¹ as set out in the preceding claims apply;
or
wherein the compound of formula (I) is a compound of formula (IIb) or a salt or solvate thereof: 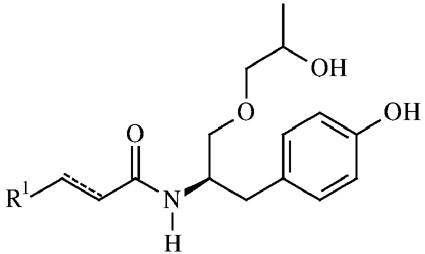 preferably selected from 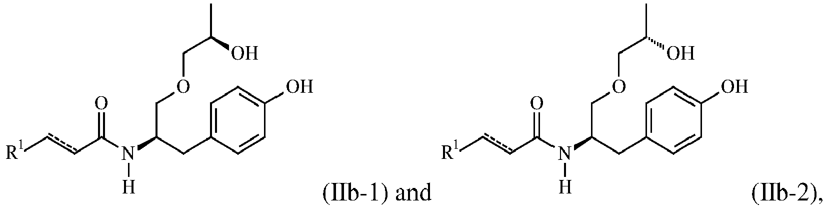 wherein the definitions of ----- and R¹ as set out in the preceding claims apply.

10. The compound for use according to any one of claims 1 to 9, wherein the compound of formula (I) is the (S,S)-isomer of 2-N(3-O-(propan-2-ol)-1-propyl-4-hydroxybenzene)-3-phenylpropylamide and has the following formula (III):

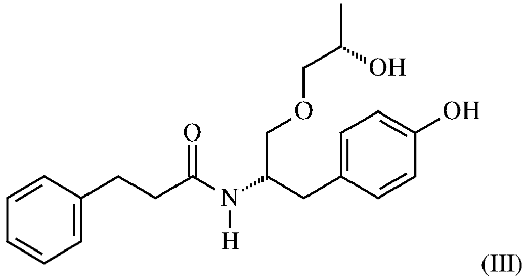

11. The compound for use according to claim 10, wherein the compound has a polymorphic form having an X-ray powder diffraction pattern (CuKa) comprising a peak at 19.0 ± 0.2 °2θ and/or a melting point in the range from about 105°C to about 110°C.

12. The compound for use of any one of claims 1 to 11, wherein the Coronavirus is of the genus α-CoV, β-CoV, γ-CoV or δ-CoV, preferably wherein the Coronavirus is selected from the group consisting of Human coronavirus OC43 (HCoV-OC43), Human coronavirus HKU1 (HCoV-HKU1), Human coronavirus 229E (HCoV-229E), Human coronavirus NL63 (HCoV-NL63, New Haven coronavirus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019") and/or wherein the Coronavirus-induced disease is selected from the group consisting of Middle East Respiratory Syndrome (MERS), Severe Acute Respiratory Syndrom (SARS) and COVID-19,
preferably wherein the coronavirus is SARS-CoV-2 and the coronavirus-induced disease is COVID-19.

13. A pharmaceutical composition for use in the treatment or prevention of an infection resulting from a Coronavirus and/or a Coronavirus-induced disease, said composition comprising a pharmaceutically effective amount of one or more of the compounds for use according to any one of claims 1 to 12, optionally together with one or more pharmaceutically acceptable excipients, optionally together with one or more further pharmaceutically active compounds.

14. The compound for use according to any one of claims 1 to 12, or the pharmaceutical composition according to claim 13, wherein said compound or pharmaceutical composition is formulated as a unit dosage form, preferably comprising from 0.1 to about 500 mg of the one or more compounds.

15. The compound for use according to any one of claims 1 to 12, or the pharmaceutical composition according to claim 13, wherein said compound or pharmaceutical composition is to be administered orally.
